# EUROPÄISCHE PATENTANMELDUNG

(11) **EP 3 441 035 A1**
(43) Veröffentlichungstag der Anmeldung: **13.02.2019**
(21) Anmeldenummer: 17185729.5
(22) Anmeldetag: 10.08.2017
(51) Int. Cl.: A61B 34/10, A61B 34/20, A61B 34/30, A61B 6/08, A61B 6/10, A61B 6/00

(54) **VISUALISIERUNGSSYSTEM ZUM ANZEIGEN EINES RAUMBEREICHES UND VERFAHREN ZUM BETREIBEN EINES VISUALISIERUNGSSYSTEMS**

(71) Anmelder: Siemens Healthcare GmbH, 91052 Erlangen (DE)
(72) Erfinder: Heigl, Benno, 96450 Coburg (DE)

(57) **Zusammenfassung**

Die Erfindung betrifft ein Visualisierungssystem (9) zum Anzeigen eines Raumbereiches (15, 18, 20) sowie ein Verfahren (21) zum Betreiben eines Visualisierungssystems (9). Dabei wird mittels einer Erfassungseinrichtung des Visualisierungssystems (9) eine Vorgabe für eine geplante oder eine maximal mögliche Bewegung eines Roboters (3) erfasst. Mittels einer von der Erfassungseinrichtung in Abhängigkeit von der Vorgabe angesteuerten Markierungseinrichtung (10, 11, 12) des Visualisierungssystems (9) wird der Raumbereich (15, 18, 20), der von dem Roboter (3) bei der Bewegung gemäß der Vorgabe erreichbar ist, optisch markiert.

## Beschreibung

Die Erfindung betrifft ein Visualisierungssystem zum Anzeigen eines Raumbereiches und ein Verfahren zum Betreiben eines Visualisierungssystems.

Mit dem Aufkommen, der zunehmenden Verbreitung und dem Einsatz von Robotern auch in solchen Arbeitsbereichen, in denen sich Menschen aufhalten oder in denen Menschen arbeiten, tritt die Problematik zutage, wie eine gleichzeitige Zusammenarbeit von Robotern und Menschen im selben Arbeitsbereich effektiv, effizient und sicher gestaltet werden kann. Es kann beispielsweise problematisch sein, einen Roboter, einen Roboterarm oder ein von einem Roboter gehaltenes Werkzeug oder Werkstück in einem solchen Arbeitsbereich zu bewegen, da die Gefahr einer Kollision mit einem Menschen besteht. Aufgrund der durch Roboter aufbringbaren Kräfte kann hier ein erhebliches Verletzungsrisiko bestehen. Dabei besteht im Gegensatz zu einer Zusammenarbeit mehrerer Personen auch die Problematik, dass Bewegungen und Lageveränderungen eines Roboters zum Erreichen einer Zielposition oftmals nicht intuitiv vorhersehbar sind. Wird andererseits der jeweilige Roboter beispielsweise nur dann betrieben oder bewegt, wenn sich kein Mensch in dem jeweiligen Gefahrenbereich aufhält, so können dadurch eine Kooperation erschwert und Arbeitsabläufe verlangsamt oder ineffizient werden. Dies kann insbesondere für zeit- und/oder sicherheitskritische Anwendungen, wie beispielsweise bei einem Einsatz von robotisch gesteuerten oder robotisch bewegten medizinischen Geräten in einem Operationssaal, problematisch sein. Als Roboter im Sinne der vorliegenden Erfindung kann ein Industrie- oder Leichtbauroboter ebenso verstanden werden wie beispielsweise ein mittels eines Antriebs gesteuert oder automatisch, also robotisch bewegbares C-Bogen Röntgengerät.

Aufgabe der vorliegenden Erfindung ist es, eine verbesserte, effiziente und sichere Kooperation zwischen Robotern und Menschen zu ermöglichen.

Diese Aufgabe wird erfindungsgemäß durch die Gegenstände der unabhängigen Patentansprüche gelöst. Vorteilhafte Ausgestaltungen und Weiterbildungen der Erfindung sind in den abhängigen Patentansprüchen sowie in der Beschreibung und in den Zeichnungen angegeben.

Ein erfindungsgemäßes Visualisierungssystem dient zum Anzeigen eines Raumbereiches. Das Visualisierungssystem weist dazu eine Erfassungseinrichtung und eine Markierungseinrichtung auf. Die Erfassungseinrichtung dient oder ist eingerichtet zum Erfassen einer Vorgabe für eine geplante und/oder eine maximal mögliche Bewegung eines Roboters. Die Markierungseinrichtung dient oder ist eingerichtet zum optischen Markieren des Raumbereiches, der von dem Roboter bei der Bewegung gemäß der Vorgabe erreichbar ist. Die Erfassungseinrichtung und die Markierungseinrichtung sind dabei direkt oder indirekt miteinander verbunden, wobei die Markierungseinrichtung von der Erfassungseinrichtung in Abhängigkeit von der Vorgabe angesteuert wird beziehungsweise angesteuert ist oder ansteuerbar ist.

Ein Raumbereich im Sinne der vorliegenden Erfindung kann dabei ein dreidimensionales Raumvolumen aber ebenso ein zweidimensionaler Flächenbereich, beispielsweise ein Teilbereich einer Oberfläche, sein. Das Anzeigen oder Markieren des Raumbereiches kann dabei beispielsweise durch Beleuchten eines Flächenbereiches oder eines Umrisses auf einer Oberfläche erfolgen. So kann beispielsweise auch ein dreidimensionales Raumvolumen durch eine zweidimensionale Markierung angezeigt oder markiert werden. Beispielsweise kann auf einem Boden eines jeweiligen Einsatzbereiches oder Raumes ein Flächenbereich oder ein Umriss markiert - also beispielsweise beleuchtet - und dadurch das sich oberhalb des markierten Flächenbereiches oder Umrisse befindende Raumvolumen angezeigt oder markiert werden. Beispielsweise durch Beleuchten einer realen Oberfläche kann also im realen Raum der Raumbereich angezeigt oder markiert werden. Zusätzlich oder alternativ kann der Raumbereich aber ebenso in oder als Teil einer augmentierten oder virtuellen Realität angezeigt oder markiert werden. Im Sinne der vorliegenden Erfindung ist das Anzeigen des Raumbereiches also sowohl im Sinne von "markieren" oder "auf etwas hinweisen" als auch im Sinne von einem Darstellen auf einer Anzeigefläche oder einem Display beziehungsweise Bildschirm zu verstehen, wobei die jeweils anzuwendende Bedeutung aus dem jeweiligen Sinnzusammenhang klar hervorgeht.

Die Vorgabe kann beispielsweise eine Benutzereingabe und/oder ein Steuersignal sein oder umfassen. Dass die Vorgabe erfasst wird und der Raumbereich in Abhängigkeit von der Vorgabe markiert wird, bedeutet dabei, dass der Raumbereich bereits vor der entsprechenden Bewegung oder sogar unabhängig von einer tatsächlichen Ausführung der entsprechenden Bewegung des Roboters markiert wird beziehungsweise markiert werden kann. Das Erfassen der Vorgabe kann ebenso ein Abrufen und/oder Empfangen von Daten aus einer Speichereinrichtung sein oder umfassen. So können beispielsweise aus einer Datenbank oder von dem Roboter Daten oder Spezifikationen abgerufen werden, welche dann zum Bestimmen des zu markierenden Raumbereiches durch das Visualisierungssystem verarbeitet werden können. Dies kann beispielsweise einen aktuellen Zustand, also beispielsweise eine aktuelle Stellung oder Pose, des Roboters ebenso umfassen wie beispielsweise einen spezifizierten maximalen Verstellumfang, der von dem Roboter erreichbar oder ausführbar ist. Ebenso kann beispielsweise ein vorgegebener Pfad abgerufen oder abgefragt werden, den der Roboter zurücklegen würde, um von seiner aktuellen Stellung in eine durch die Vorgabe vorgegebene Zielstellung zu gelangen. Für einen derartigen Pfad können je nach Ausgestaltung des Roboters mehrere Möglichkeiten bestehen, wobei die tatsächliche Bewegung des Roboters beispielsweise durch ein Robotersteuergerät und nicht durch das erfindungsgemäße Visualisierungssystem bestimmt sein oder bestimmt werden kann. Selbstverständlich können die entsprechenden Funktionalitäten aber ebenso in einer Vorrichtung oder in einem System integriert sein, sodass das Visualisierungssystem beispielsweise Teil eines Robotersystems sein kann, welches auch den Roboter selbst und dessen Steuerung umfasst. Wird das Visualisierungssystem separat von dem Roboter ausgebildet, so kann dieses vorteilhaft beispielsweise kosteneffizient mit einer höheren Rechenleistung ausgestattet und/oder für mehrere Roboter verwendet werden. Bei einer Integration des Visualisierungssystems mit dem Roboter oder der Robotersteuerung, kann das Visualisierungssystem vorteilhaft besonders einfach und genau an die Spezifikationen des jeweiligen Roboters angepasst werden. Zudem kann der Roboter beispielsweise räumlich und/oder unabhängig von einer externen Infrastruktur zur Anbindung an das Visualisierungssystem und/oder zur Datenübertragung betrieben werden.

Die Vorgabe kann beispielsweise von einem jeweiligen Benutzer durch Angeben einer bestimmten Zielstellung für den Roboter gemacht oder angeben werden. Diese Vorgabe wird dann erfasst und der entsprechende Raumbereich, der von dem Roboter bei der tatsächlichen Ausführung der Bewegung in zu der vorgegebenen Zielposition erreicht, also etwa durchlaufen oder überfahren, wird beziehungsweise würde, wird automatisch markiert. Betrifft die Vorgabe die maximal mögliche Bewegung des Roboters, so kann beispielsweise der Raumbereich markiert werden, der bei Ausnutzung des maximalen Verstellumfangs des Roboters von diesem erreicht wird beziehungsweise erreicht werden kann. Dabei kann lediglich ein Raumbereich einer vorgegebenen Größe markiert werden, der beispielsweise einem am weitesten von einer Aufhängung, einem Lagerpunkt oder einer Neutral- oder Ruhestellung des Roboters entfernt ist. Ebenso kann jedoch auch hierbei der gesamte von dem Roboter bei der entsprechenden Bewegung von seiner aktuellen Stellung in eine maximal verstellte oder ausgelenkte Stellung durchlaufen, überfahren oder überstrichen würde.

Die Erfassungseinrichtung kann also beispielsweise eine Schnittstelle oder ein Interface, insbesondere ein Benutzerinterface, sein oder umfassen. Über dieses kann das Visualisierungssystem die Vorgabe beziehungsweise entsprechende Signale oder Daten empfangen. Ebenso kann ein den Roboter bewegendes und/oder ansteuerndes System oder Gerät die Vorgabe und/oder entsprechende Daten der Erfassungseinrichtung bereitstellen, beispielsweise durch ein Benutzerinterfaces dieses Systems oder Gerätes und/oder durch Zustandsdaten, die einen aktuellen Zustand des Systems oder Gerätes beschreiben oder angeben. Die Erfassungseinrichtung oder das Visualisierungssystem können ebenso eine Datenverarbeitungseinrichtung zum Verarbeiten der erfassten Vorgabe beziehungsweise zum Abrufen von zum Bestimmen des Raumbereiches und/oder zum Markieren des Raumbereiches notwendigen oder hilfreichen Daten umfassen. Die Erfassungseinrichtung beziehungsweise die Datenverarbeitungseinrichtung kann ebenso zum Ausführen oder Ausüben von Steuerfunktionen für die Markierungseinrichtung und/oder für den Roboter eingerichtet sein. Dazu kann die Erfassungseinrichtung beziehungsweise die Datenverarbeitungseinrichtung beispielsweise entsprechenden Programmcode und wenigstens einen zum Ausführen dieses Programmcodes eingerichteten Mikro- oder Computerchip aufweisen.

Die Markierungseinrichtung kann zum Beispiel eine oder mehrere Leuchten, beispielsweise Laser oder ein Laserarray, aufweisen. Zusätzlich oder alternativ kann die Markierungseinrichtung beispielsweise ein Leuchtband aus mehreren nebeneinander angeordneten Lichtquellen aufweisen, die einzeln oder in Gruppen, also abschnitts- oder segmentweise, angesteuert werden können. Ebenso kann die Markierungseinrichtung zusätzlich oder alternativ eine Projektionseinrichtung aufweisen, mittels welcher eine Markierung zum Markieren des Raumbereiches projizierbar ist. Wie bereits angedeutet, kann die Markierungseinrichtung zusätzlich oder alternativ beispielsweise ein Display oder einen Bildschirm, insbesondere ein Head-Mounted Display (HMD) aufweisen. Ist es vorgesehen, dass zum Markieren des Raumbereiches ein derartiges HMD verwendet wird beziehungsweise verwendet werden soll, so kann als die oder als Teil der Markierungseinrichtung ebenso eine Ausgabeeinrichtung einschließlich einer Ausgabeschnittstelle verstanden werden, welche zum Übermitteln entsprechender Steuersignale für und an das HMD dient beziehungsweise eingerichtet ist. Diese Steuersignale können das HMD dann dazu veranlassen, den Raumbereich zu markieren oder entsprechend darzustellen.

Beispielsweise kann es während einer medizinischen Prozedur notwendig sein, einen Patienten mit einem C-Bogen-Röntgengerät aus mehreren unterschiedlichen Perspektiven zu röntgen. Muss dazu der C-Bogen verfahren, also bewegt werden, so kann vor der eigentlichen Bewegung der als Verfahrweg für den C-Bogen freizuhaltende Raumbereich markiert werden. Hierdurch kann beispielsweise ein behandelnder Radiologe besonders schnell und eindeutig feststellen, ob der markierte Raumbereich frei von Gegenständen, Personen oder Hindernissen ist, sodass der C-Bogen dann schnellstmöglich in seine neue Zielstellung verfahren werden kann. Durch das Markieren des Raumbereiches vor der eigentlichen Bewegung kann sich zudem auch weiteres anwesendes Personal vorteilhaft selbstständig aus dem entsprechend markierten Raumbereich herausbewegen, ohne dass hierfür zeitaufwendige und ablenkende verbale Aufforderungen notwendig wären. So kann durch die vorliegende Erfindung also die Prozedur beziehungsweise deren Ablauf (Workflow) besonders schnell, effizient und sicher durchgeführt werden.

Ebenso ist es möglich, dass vor einer Bewegung des C-Bogens durch eine entsprechende Vorgabe - beispielsweise des behandelnden Radiologen - derjenige Raumbereich markiert wird, der bei maximaler Verstellung oder Auslenkung des C-Bogen mittels dessen Röntgenstrahlung abgebildet oder erfasst werden kann. So kann beispielsweise ein entsprechender maximal erreichbarer Aufnahmebereich auf oder an einem Patientenlager und/oder dem Patienten selbst durch entsprechende Beleuchtung, also optisch, markiert werden. So kann vorteilhaft besonders einfach und schnell festgestellt werden, ob ein abzubildender Bereich des Patienten bei der jeweils aktuellen Anordnung oder Konfiguration des Patientenlagers beziehungsweise des Patienten relativ zu dem C-Bogen tatsächlich abgebildet werden kann oder ob beispielsweise der Patient in eine andere Lage oder Stellung gebracht werden muss. Durch die vorliegende Erfindung kann dabei ein bisher oftmals übliches zeitaufwendiges Ausprobieren entfallen, bei dem beispielsweise der C-Bogen verstellt oder verfahren wird und erst dann festgestellt werden kann, ob der abzubildende Bereich des Patienten tatsächlich abgebildet werden kann. Auch durch das Markieren des bei einer maximal möglichen Bewegung des Roboters erreichbaren Raumbereiches kann also der jeweilige Ablauf verbessert, insbesondere schneller und sicherer durchgeführt werden. Insgesamt können durch die vorliegende Erfindung vorteilhaft unnötige Neupositionierungen des Roboters und/oder eines Zielobjektes, beispielsweise eines Werkstückes oder des Patienten, vermieden und tatsächlich ausgeführte Bewegungen des Roboters mit erhöhter Sicherheit und Effizienz durchgeführt werden. Es kann sich beispielsweise die Situation ergeben, dass für eine Aufnahme eines 3D-Datensatzes des Patienten der C-Bogen beziehungsweise die jeweilige Strahlquelle und der zugehörige Detektor um den Patienten herum bewegt werden muss. In einem solchen Fall kann durch die vorliegende Erfindung die Lagerung des Patienten besonders einfach, zuverlässig und mit möglichst geringer Belastung für den Patienten vorgenommen werden, da durch die optische Markierung bereits vor der entsprechenden Bewegung des C-Bogens erkennbar ist, ob diese kollisionsfrei um den abzubildenden Bereich durchgeführt werden kann.

Besonders vorteilhaft kann der jeweilige Raumbereich dynamisch markiert beziehungsweise visualisiert werden. Ein dynamisches Markieren des Raumbereiches bedeutet dabei, dass in Echtzeit eine Anpassung oder Aktualisierung des markierten Raumbereiches in Abhängigkeit von einer jeweils aktuellen Stellung und/oder Bewegung und/oder in Abhängigkeit von einem aktuellen Betriebszustand des Roboters erfolgt. Beispielsweise kann auf eine bestimmte Vorgabe für eine geplante Bewegung hin ein erster Raumbereich markiert werden. Wird die entsprechende Bewegung gestartet, dann jedoch während der Bewegung der Roboter abweichend von der Vorgabe beziehungsweise der entsprechenden geplanten Bewegung bewegt oder gesteuert, so kann durch das dynamische Markieren ein neuer, also zweiter Raumbereich markiert werden, der für die jeweils aktuelle, von der Vorgabe abweichende Bewegung relevant ist. Die Markierung des ersten Raumbereiches kann dann selbst verständlich aufgehoben oder ausgeblendet werden. So kann also beispielsweise stets ein Raumbereich in Bewegungsrichtung des Roboters vor diesem markiert werden - und zwar insbesondere auch dann, wenn der Roboter seine Bewegungsrichtung ändert.

Besonders vorteilhaft kann die vorliegende Erfindung auch für Planungszwecke oder als beziehungsweise als Teil eines Planungssystems verwendet werden. So kann beispielsweise durch Markieren oder Visualisieren der bei verschiedenen Bewegungen des Roboters von diesem erreichbaren Raumbereiche eine optimale Positionierung des Roboters in dem jeweiligen Raum bei dessen Gestaltung bestimmt werden. Ebenso können beispielsweise komplexe Abläufe, beispielsweise mehrschrittige Operationen, besonders einfach, effizient und anschaulich vor ihrer Durchführung geplant werden, da lediglich die entsprechenden Raumbereiche die zu einem bestimmten Zeitpunkt oder in einem bestimmten Zeitraum freizuhalten und/oder erreichbar sind, markiert beziehungsweise visualisiert werden können, ohne den jeweiligen Roboter tatsächlich bewegen zu müssen. Eine solche Planung kann dabei in dem jeweiligen realen Raum sowie zusätzlich oder alternativ in einer augmentierten oder virtuellen Realität durchgeführt werden.

Gerade bewegliche Roboter, welche beispielsweise mehrere Gelenke aufweisen, können aus einer jeweils aktuellen Stellung heraus eine vorgegebene Zielposition oftmals auf unterschiedlichen Pfaden oder mit unterschiedlichen Bewegungen erreichen. Die vorliegende Erfindung kann dann besonders vorteilhaft dazu genutzt werden, um - beispielsweise mittels unterschiedlicher Farben und/oder Muster - mehrere Alternativen für mögliche Bewegungen oder Pfade durch Markieren oder Anzeigen der entsprechenden dabei jeweils überstrichenen oder durchlaufenen Raumbereiche anzuzeigen beziehungsweise zu verdeutlichen oder zu visualisieren. So kann vorteilhaft in einer gegebenen Situation eine bestimmte, beispielsweise eine am besten geeignete oder einen geringsten Aufwand für ein Freiräumen des jeweiligen Raumbereiches benötigende, Alternative ausgewählt werden. Dies kann besonders vorteilhaft sein, da Bewegungen eines komplexen Roboters mit beispielsweise wenigstens sechs Freiheitsgraden beziehungsweise wenigstens sechs Achsen, insbesondere für fachfremdes Personal, keineswegs intuitiv zuverlässig vorhersehbar sind.

Zum Markieren der alternativ möglichen Bewegungen oder Pfade können automatisch beispielsweise eine aktuelle Konfiguration oder Pose des Roboters ebenso berücksichtigt werden, wie dessen Ausstattung, also dessen Verstellmöglichkeiten und/oder beispielsweise eine aktuell von dem Roboter gehaltene und/oder eine an der jeweiligen Zielposition von dem Roboter aufzunehmende Last. Die möglichen Alternativen können dabei beispielsweise von dem Roboter beziehungsweise von dessen Steuersystem selbst oder von dem erfindungsgemäßen Visualisierungssystem bestimmt werden. Dazu notwendige Daten, wie beispielsweise eine Spezifikation des Roboters, können dazu beispielsweise vorab bereitgestellt sein oder von dem Roboter oder aus einer bereitgestellten Datenbank abgerufen werden.

Wird dann beispielsweise durch eine entsprechende Bedienhandlung eine der Alternativen ausgewählt beziehungsweise eine entsprechende Bedienhandlung oder Benutzereingabe erfasst, so kann die Markierung des oder der alternativen Raumbereiche aufgehoben oder ausgeblendet werden. Auch kann der Roboter dann automatisch entsprechend der ausgewählten Alternative gesteuert beziehungsweise angesteuert werden.

In vorteilhafter Ausgestaltung der vorliegenden Erfindung weist die Markierungseinrichtung eine Leuchte auf, in der eine Lichtquelle zur flächigen Ausleuchtung eines Arbeitsbereiches des Roboters mit inkohärentem Licht und eine Laserlichtquelle zum Markieren des Raumbereiches angeordnet sind. Die Leuchte kann beispielsweise ein Flutlicht oder eine OP-Leuchte für einen Operationssaal sein. Durch die inkohärente Lichtquelle kann vorteilhaft der jeweilige Einsatz- oder Arbeitsbereich besonders gleichmäßig ausgeleuchtet werden, während mittels der Laserlichtquelle aufgrund ihrer Intensität und Strahlcharakteristik trotz der Ausleuchtung des Arbeitsbereiches durch die inkohärente Lichtquelle der Raumbereich mit geringem Aufwand besonders zuverlässig und besonders gut wahrnehmbar markiert werden kann. Die Kombination dieser beiden Lichtquellen in einer einzigen Leuchte ist besonders vorteilhaft, da zum Ausleuchten des Arbeitsbereiches die in kohärente Lichtquelle ohnehin stets so positioniert ist beziehungsweise wird, dass der jeweilige Arbeitsbereich ohne Verdeckungen oder Schattenwürfe ausgeleuchtet ist beziehungsweise ausgeleuchtet werden kann. Somit kann auch der Raumbereich mittels der Laserlichtquelle aufgrund von deren Anordnung an oder in der Leuchte beziehungsweise an der inkohärentem Lichtquelle ohne zusätzlichen Aufwand oder zusätzliche Vorkehrungen möglichst verdeckungsfrei, insbesondere im Blickfeld von jeweiligem anwesendem Personal, markiert werden. Die Laserlichtquelle kann beispielsweise zusammen mit der inkohärenten Lichtquelle beziehungsweise mehreren derartigen inkohärenten Lichtquellen in einem gemeinsamen Gehäuse und/oder an einer gemeinsamen Halterung angeordnet sein, wodurch vorteilhaft ein gemeinsames Bewegen oder Verstellen beider Lichtquellen ermöglicht werden kann. Die Leuchte beziehungsweise die Laserlichtquelle kann zusätzlich eine Ablenkungsoptik zum Ablenken eines von der Laserlichtquelle ausgesendeten Laserstrahls aufweisen. Durch eine derartige Optik kann der Laserstrahl zum Markieren des Raumbereiches abgelenkt werden. So kann der Laserstrahl beispielsweise einem Umriss oder einer Kontur des zu markierenden Raumbereiches folgen.

In vorteilhafter Ausgestaltung der vorliegenden Erfindung weist die Markierungseinrichtung eine Laserlichtquelle zum zumindest bereichsweisen Einhüllen eines Zielobjekts des Roboters mittels von der Laserlichtquelle ausgestrahltem Laserlicht auf. Diese Laserlichtquelle kann die vorliegend bereits an anderer Stelle erwähnte Laserlichtquelle oder eine zweite Laserlichtquelle sein. Das Zielobjekt kann beispielsweise ein von dem Roboter bearbeitetes oder zu bearbeitendes Werkstück oder ein Patient sein, der beispielsweise mittels eines robotischen oder robotisch bewegten Röntgengeräts abgebildet werden soll. Zum Einhüllen des Zielobjekts mittels des Laserlichts kann dieses beispielsweise in einem vorgegebenen Abstand zu dem Zielobjekt zumindest im Wesentlichen parallel zu dem Zielobjekt und/oder parallel oder tangential zu einer gedachten, beispielsweise quader-, kegel- oder zylinderförmigen, das Zielobjekt zumindest bereichsweise umgebenden Mantel- oder Hüllfläche ausgestrahlt oder gelenkt werden. Ragt dann ein Objekt durch diese Hüllfläche hindurch, so wird es von dem Laserlicht in einem entsprechenden Durchtrittsbereich, in dem das Objekt die gedachte Hüllfläche schneidet, angeleuchtet oder angestrahlt. So kann beispielsweise eindeutig und besonders einfach und schnell und mit besonders geringem Aufwand festgestellt werden, ob ein für eine ungehinderte Bewegung des Roboters um das Zielobjekt oder entlang des Zielobjektes frei von Hindernissen ist.

Beispielsweise kann es während einer medizinischen Prozedur vorgesehen sein, dass eine Strahlenquelle und/oder ein Detektor eines Röntgengerätes in einem vorgegebenen Abstand, beispielsweise in einem Abstand von 30 cm, zu dem Zielobjekt um dieses herum oder an diesem entlang verfahren werden soll. Während derselben Prozedur kann ein medizinisches Objekt, beispielsweise eine Klammer, eine Positionierhilfe, ein K-Draht oder dergleichen, an dem Patienten, beispielsweise an der Wirbelsäule des Patienten, befestigt sein und in eine Umgebung des Patienten hineinragen. Es ist dann unmittelbar ersichtlich, ob eine Kollisionsgefahr zwischen dem jeweiligen medizinischen Objekt und dem Röntgengerät besteht, da in einem solchen Fall das medizinische Objekt durch das Laserlicht angestrahlt beziehungsweise markiert wird. So kann durch die vorliegende Erfindung also beispielsweise ein bisher oftmals üblicher Probelauf des Röntgengeräts und/oder eine gegebenenfalls notwendige Neupositionierung des Patienten vermieden werden. Das Zielobjekt kann dabei lediglich teilweise oder bereichsweise von dem Laserlicht eingehüllt werden, wenn beispielsweise nicht alle Oberflächenbereiche des Zielobjekts zugänglich oder exponiert sind und/oder wenn der Roboter beispielsweise nur über einen bestimmten Teilbereich des Zielobjekts bewegt werden soll.

In vorteilhafter Ausgestaltung der vorliegenden Erfindung weist die Markierungseinrichtung eine Darstellungseinrichtung, insbesondere ein Head-Mounted Display (HMD), zum Visualisieren des Raumbereiches mittels einer auf augmentierter Realität basierenden Darstellung auf. Die Markierungseinrichtung ist mit anderen Worten also dazu eingerichtet, den Raumbereich virtuell auf einer Anzeigefläche oder einem Display der Darstellungseinrichtung anzuzeigen oder zu markieren. Durch dieses virtuelle beziehungsweise augmentierte Visualisieren des Raumbereiches kann vorteilhaft insbesondere ein dreidimensionaler Raumbereich, also ein Raumvolumen, vollständig räumlich markiert werden. Dies kann vorteilhaft zu einer besseren Erkennbarkeit oder Erkennungsrate von Kollisionsgefahren und somit zu einer verbesserten Sicherheit beim Betreiben des Roboters führen beziehungsweise beitragen. Besonders vorteilhaft kann der Raumbereich dabei unabhängig von einer Anordnung externer, also nicht an dem HMD angeordneter, Lichtquellen aus beliebigen Blickwinkeln markiert, also visualisiert werden. Ein jeweiliger Nutzer der Darstellungseinrichtung, also beispielsweise ein jeweiliger Träger des HMD, kann dabei vorteilhaft unabhängig von seiner eigenen Stellung und/oder unabhängig von möglichen Sichtverdeckungen, beispielsweise durch andere Personen oder Geräte, den jeweiligen Raumbereich erkennen, insbesondere ohne dass er hierfür eine Bedienhandlung ausführen oder beispielsweise seine Hände einsetzen müsste.

In vorteilhafter Ausgestaltung der vorliegenden Erfindung weist das Visualisierungssystem eine 3D-Kamera zum räumlichen Erfassen einer, insbesondere den Raumbereich umfassenden, Umgebung des Roboters auf. Die 3D-Kamera stellt dann entsprechende Umgebungsdaten bereit, die eine räumliche Abbildung oder Repräsentation der erfassten Umgebung umfassen. Weiterhin ist das Visualisierungssystem dann eingerichtet zum Markieren des Raumbereiches in Abhängigkeit von den von der 3D-Kamera bereitgestellten Umgebungsdaten. Mit anderen Worten können die Umgebungsdaten beziehungsweise eine durch diese beschriebene oder charakterisierte räumliche Konfiguration der Umgebung, das heißt also eine räumliche Anordnung von Objekten und/oder Personen in der erfassten Umgebung, zum Bestimmen des zu markierenden Raumbereiches und/oder zum Steuern der Markierungseinrichtung berücksichtigt werden. Beispielsweise kann eine perspektivische oder geometrische Verzerrung durch in der erfassten Umgebung angeordnete Objekte und/oder aus einer jeweiligen Perspektive oder einem jeweiligen Blickwinkel der in der Umgebung anwesenden Personen berücksichtigt und das Markieren des Raumbereiches entsprechend angepasst werden.

Ebenso kann beispielsweise durch Auswerten der Umgebungsdaten automatisch ermittelt werden, welche Objekte oder Personen sich in dem zu markierenden Raumbereich befinden. Diese Objekte oder Personen können dann beispielsweise zusätzlich markiert werden, beispielsweise durch eine flächige Beleuchtung des jeweiligen Objekts oder der jeweiligen Person. Hierdurch kann das jeweilige Objekt beziehungsweise die jeweilige Person besonders deutlich markiert und somit eine entsprechende Kollisionsgefahr mit dem Roboter besonders gut, einfach und zuverlässig erkennbar angezeigt werden. Ebenso kann beispielsweise ein ganz oder teilweise in dem Raumbereich angeordnetes Objekt an einer Stelle durch die Markierungseinrichtung markiert werden, die sich aktuell oder mit größerer Wahrscheinlichkeit in einem Blickfeld einer anwesenden Person befindet. Auch hierdurch kann die Erkennbarkeit beziehungsweise die Erkennungsrate von Kollisionsgefahren und somit die Sicherheit beim Betrieb des Roboters verbessert werden.

Ein erfindungsgemäßes Verfahren dient zum Betreiben eines Visualisierungssystems, insbesondere eines erfindungsgemäßen Visualisierungssystems. Bei dem Verfahren wird eine Vorgabe für eine geplante und/oder für eine maximal mögliche Bewegung eines Roboters erfasst. Anschließend wird ein Raumbereich, der von dem Roboter bei der Bewegung erreichbar ist, optisch markiert. Da hierfür insbesondere das erfindungsgemäße Visualisierungssystem verwendet werden kann, gelten die im Zusammenhang mit diesem erläuterten Ausgestaltungen auch für das vorliegende Verfahren, weshalb diese der Übersichtlichkeit halber hier nicht noch einmal explizit in aller Vollständigkeit beschrieben sind.

In einer vorteilhaften Ausgestaltung der vorliegenden Erfindung wird als der Raumbereich ein für eine ungehinderte Bewegung des Roboters freizuhaltender Raumbereich markiert. Der freizuhaltende Raumbereich ist dabei ein Raumbereich, der von dem Roboter bei der Bewegung von einer aktuellen Istposition des Roboters hin zu einer vorgegebenen Zielposition durchlaufen und/oder überfahren wird beziehungsweise würde. Mit anderen Worten wird also derjenige Raumbereich markiert, in dem bei einer Ausführung der entsprechenden Bewegung des Roboters eine Kollisionsgefahr besteht.

In weiterer vorteilhafter Ausgestaltung der vorliegenden Erfindung wird als der Raumbereich ein von dem als medizinisches bildgebendes Gerät ausgebildeten Roboter, insbesondere bei Ausnutzung eines maximal möglichen Verstell- oder Bewegungsumfangs des Roboters, abbildbarer Bereich eines Untersuchungsobjekts, beispielsweise eines Patienten, optisch markiert. Dieser abbildbare Bereich kann also - beispielsweise wenn es sich bei dem Roboter um ein robotisch bewegtes oder bewegbare C-Bogen-Röntgengerät handelt - von einer von dem Röntgengerät ausgesendeten und anschließend detektierten Röntgenstrahlung durchdrungen, also erreicht werden.

Im Sinne der vorliegenden Erfindung sind daher nicht nur Raumbereiche, die von einem materiellen Bestandteil oder Element des Roboters erreicht werden können als von dem Roboter erreichbare Raumbereiche anzusehen. Vielmehr gelten auch solche Raumbereiche als von dem Roboter erreichbar, die sich beispielsweise in einem Erfassungs-, Abbildung- und/oder Aufnahmebereich und/oder in einem Wirk- oder Manipulationsbereich, der von dem Roboter bearbeitet, beeinflusst oder manipuliert werden kann, befinden.

In weiterer vorteilhafter Ausgestaltung der vorliegenden Erfindung wird in Abhängigkeit von der Vorgabe eine bei der entsprechenden Bewegung des als medizinisches bildgebendes Gerät ausgebildeten Roboters auftretende räumliche Verteilung einer Strahlendosis berechnet. In dem markierten Raumbereich und/oder in einer Umgebung des Raumbereiches wird dann die jeweils berechnete, absolute oder relative, Strahlendosis visualisiert, insbesondere durch unterschiedliche Kennzeichnung von Zonen, in denen die jeweilige Strahlendosis in unterschiedlichen vorgegebenen Werteintervallen liegt. Mit anderen Worten wird also bestimmt, wie hoch eine Strahlendosis oder Strahlenbelastung in einem oder mehreren räumlichen Bereichen beziehungsweise Zonen ist beziehungsweise wäre, wenn die der Vorgabe entsprechende Bewegung des Roboters tatsächlich ausgeführt wird oder würde. Die entsprechende Strahlendosis kann dann beispielsweise farbcodiert angezeigt oder visualisiert werden. Beispielsweise kann eine rote Farbe oder Markierung eine höhere Strahlendosis anzeigen als beispielsweise eine gelbe oder grüne Farbe oder Markierung. Hierdurch kann anwesendes Personal nicht nur eine Kollision mit dem Roboter durch Verlassen des markierten Raumbereiches vermeiden, sondern auch selbstständig eine jeweilige Strahlenbelastung minimieren, beispielsweise durch Auswahl einer Ausweichposition mit minimaler Strahlenbelastung oder Strahlendosis.

Beispielsweise kann es sich bei dem Roboter um ein Röntgengerät handeln, dessen Röntgenstrahlung bauart- und/oder situationsbedingt auch außerhalb des Untersuchungsobjekts angeordnete Raumbereiche durchdringen beziehungsweise durchstrahlen kann. Durch die somit ermöglichte Minimierung der Strahlendosis kann die Sicherheit beim Betrieb des Roboters weiter verbessert werden. Auch eine Strahlenbelastung von Geräten oder Material kann so besonders einfach, effektiv und effizient reduziert werden, da aufgrund der optischen Markierung beispielsweise besonders einfach erkannt werden kann, ob sich ein bewegliches beziehungsweise belegbares Objekt in einem Bereich projizierter beziehungsweise vorhergesagter relativ hoher Strahlenbelastung oder Strahlendosis befindet und/oder ob das Objekt - beispielsweise mit einem Handgriff - in einen Bereich mit geringerer projizierter oder vorhergesagter Strahlenbelastung bewegt werden kann. Dies kann beispielsweise empfindliches biologisches Material, wie etwa Zellkulturen oder Ersatzorgane, betreffen.

Durch die Kennzeichnung unterschiedlicher Zonen gemäß den vorgegebenen Werteintervallen kann vorteilhaft auch für Laien eine schnelle, sichere und intuitive Verständlichkeit der jeweiligen Strahlendosis in der jeweiligen Zone, also in dem jeweiligen räumlichen Bereich, erreicht werden.

In weiterer vorteilhafter Ausgestaltung der vorliegenden Erfindung kann eine in Abhängigkeit von der Vorgabe eine bei der entsprechenden Bewegung des Roboters auftretende räumliche Verteilung einer Immission berechnet. In dem markierten Raumbereich und/oder in einer Umgebung des Raumbereiches wird dann die jeweils berechnete, absolute oder relative, Immission visualisiert, insbesondere durch unterschiedliche Kennzeichnung von Zonen, in denen die jeweilige Immission in unterschiedlichen vorgegebenen Werteintervallen liegt. Mit anderen Worten wird also bestimmt, wie hoch eine Immission oder Immissionsbelastung oder -dosis in einem oder mehreren räumlichen Bereichen beziehungsweise Zonen ist beziehungsweise wäre, wenn die der Vorgabe entsprechende Bewegung des Roboters tatsächlich ausgeführt wird oder würde. Die entsprechende Immission kann dann beispielsweise farbcodiert angezeigt oder visualisiert werden. Beispielsweise kann eine rote Farbe oder Markierung eine höhere Immission anzeigen als beispielsweise eine gelbe oder grüne Farbe oder Markierung. Hierdurch kann anwesendes Personal nicht nur eine Kollision mit dem Roboter durch Verlassen des markierten Raumbereiches vermeiden, sondern auch selbstständig eine jeweilige Belastung durch die jeweilige Immission - beziehungsweise die entsprechende Emission des Roboters und/oder eines von dem Roboter durchgeführten Arbeitsprozesses - minimieren, beispielsweise durch Auswahl einer Ausweichposition mit minimaler Immission. Eine Immission in diesem Sinne kann beispielsweise ein Eintrag von Lärm, Material - wie beispielsweise Spänen oder Funken - und/oder Geruch oder Abgas in den jeweiligen Raumbereich oder in die jeweilige Zone sein oder bedeuten. Diese kann insbesondere auftreten, wenn der Roboter als Industrieroboter ausgebildet ist.

In weiterer vorteilhafter Ausgestaltung der vorliegenden Erfindung wird eine den Raumbereich umfassende Umgebung des Roboters automatisch räumlich erfasst und auf ein Vorhandensein von Hindernissen hin überwacht. Wenn ein Hindernis in dem Raumbereich oder in der Umgebung detektiert wird, wird automatisch eine Warnung ausgegeben und/oder die Bewegung des Roboters in Abhängigkeit von einer detektierten Position des Hindernisses angepasst. Zum Überwachen der Umgebung kann diese beispielsweise mittels einer 3D-Kamera und/oder eines Bewegungssensors erfasst werden. Zum Detektieren des Hindernisses können die von der 3D-Kamera bereitgestellten Umgebungsdaten beispielsweise mittels einer Bildverarbeitungseinrichtung ausgewertet oder verarbeitet werden. Hierbei kann bevorzugt ein Objekterkennungsalgorithmus und/oder ein neuronales Netz eingesetzt werden.

Es ist beispielsweise möglich, das Hindernis - also beispielsweise ein Objekt oder eine Person - in der Umgebung zu detektieren, wenn es sich noch außerhalb des markierten oder zu markierenden Raumbereiches befindet. Das beziehungsweise ein detektiertes Hindernis beziehungsweise dessen Position kann kontinuierlich nachverfolgt werden (Object-Tracking). Aus einer hieraus bestimmten Bewegungsrichtung des Hindernisses und/oder einer automatisch durchgeführten Extrapolation einer detektierten Bewegung des Hindernisses kann beispielsweise ein Wahrscheinlichkeitswert dafür berechnet werden, dass sich das Hindernis in den Raumbereich hinein bewegt. Dann kann die Warnung beispielsweise ausgegeben werden, wenn der berechnete Wahrscheinlichkeitswert einen vorgegebenen Schwellenwert erreicht oder überschreitet. So kann vorteilhaft die Warnung bereits ausgegeben werden, bevor das Hindernis den Raumbereich erreicht und somit bevor eine akute Kollisionsgefahr mit dem Roboter besteht.

Die Warnung kann eine optische Warnung, also beispielsweise ein optisches Signal sein oder umfassen. So kann beispielsweise eine Intensität oder Helligkeit der Markierung, mit der der Raumbereich markiert ist, erhöht oder beispielsweise in einer vorgegebenen Art variiert werden. So kann die Markierung als Warnung blinkend statt kontinuierlich angezeigt werden. Zusätzlich oder alternativ kann eine Farbe und/oder ein Muster oder/oder eine Form der Markierung als Warnung verändert werden. Ebenso kann die Warnung zusätzlich oder alternativ eine akustische Warnung, also ein akustisches Signal sein oder umfassen.

Besonders vorteilhaft können mehrere vorgegebene Eskalationsstufen für die Warnung vorgesehen sein, welche mit unterschiedlichen Warnsignalen einhergehen oder verknüpft sein können. Die unterschiedlichen Eskalationsstufen können beispielsweise in Abhängigkeit von dem berechneten Wahrscheinlichkeitswert und/oder in Abhängigkeit von der jeweils aktuellen Position des Hindernisses und/oder des Roboters durchlaufen werden. So kann beispielsweise die Warnung in eine höhere Warnstufe eskaliert werden, wenn sich der Roboter in Bewegung setzt und/oder wenn beispielsweise ein Abstand zwischen dem Roboter und dem Hindernis einen vorgegebenen Abstandsschwellenwert erreicht oder unterschreitet.

Das Anpassen der Bewegung des Roboters kann beispielsweise ein Verlangsamen oder Stoppen der Bewegung bedeuten. Ebenso kann es aber möglich sein, beispielsweise automatisch den Pfad und/oder die Konfiguration oder Pose des Roboters derart zu verändern, dass er dem detektierten Hindernis ausweicht und dennoch die vorgegebene Zielposition erreicht. Hierzu kann die Robotersteuerung beispielsweise mit dem Visualisierungssystem und/oder der Datenverarbeitungseinrichtung des Visualisierungssystems mittels einer Datenverbindung verbunden sein.

Beispielsweise können Verstellmöglichkeiten oder eine Gelenkigkeit des Roboters ausgenutzt werden, um dem detektierten Hindernis auszuweichen. Ob die Bewegung des Roboters gestoppt oder zum Ausweichen verändert wird, kann beispielsweise in Abhängigkeit davon automatisch bestimmt werden, ob der Roboter eine auszuführende Aufgabe, beispielsweise einen Scan eines Untersuchungsobjekts, trotz der möglichen Veränderung oder Anpassung der Bewegung ausführen kann. Beispielsweise können zum erfolgreichen Ausführen des Scans lediglich eine Position und ein Pfad einer Strahlquelle und eines Detektors relevant sein, während eine genaue Stellung von einzelnen Abschnitten oder Gliedern eines Roboterarms des Roboters irrelevant sein kann.

Wird die Bewegung oder der Pfad des Roboters verändert, so kann, bevorzugt in Echtzeit, auch die Markierung des Raumbereiches entsprechend angepasst, also aktualisiert werden. Beispielsweise kann bei einer Änderung des Pfades des Roboters die bisherige Markierung aufgehoben oder ausgeblendet und stattdessen ein neuer oder aktualisierter Raumbereich markiert werden, der von dem Roboter gemäß dem neuen, also geänderten Pfad erreicht wird beziehungsweise erreichbar ist.

Insgesamt kann durch diese Maßnahmen die Effizienz und Sicherheit beim Betreiben des Roboters weiter verbessert werden, da unnötige Wartezeiten und Kollisionen oder Kollisionsgefahren vermieden oder minimiert werden können.

Das erfindungsgemäße Visualisierungssystem kann insbesondere zum Ausführen zumindest einer Ausführungsform des erfindungsgemäßen Verfahrens eingerichtet sein. Dazu kann das Visualisierungssystem beispielsweise einen Datenträger oder ein Speichermedium mit einem jeweilige Verfahrensschritte des erfindungsgemäßen Verfahrens beschreibenden oder kodierenden Programmcode umfassen. Ebenso kann das Visualisierungssystem eine Steuer- oder Datenverarbeitungseinrichtung mit einer Prozessoreinheit zum Ausführen dieses Programmcodes aufweisen.

Die bisher und im Folgenden angegebenen Eigenschaften und Weiterbildungen des erfindungsgemäßen Visualisierungssystems und des erfindungsgemäßen Verfahrens sowie die entsprechenden Vorteile sind jeweils sinngemäß wechselseitig zwischen dem erfindungsgemäßen Visualisierungssystem und dem erfindungsgemäßen Verfahren übertragbar. Es gehören also zu der vorliegenden Erfindung auch solche Weiterbildungen des erfindungsgemäßen Visualisierungssystems und des erfindungsgemäßen Verfahrens, welche Ausgestaltungen aufweisen, die hier nicht explizit in der jeweiligen Kombination beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigen:
- FIG 1: eine schematische Draufsicht auf eine Behandlungssituation, in der ein Patient mittels eines robotisch bewegten C-Bogen-Röntgengeräts untersucht wird; und
- FIG 2: einen schematischen beispielhaften Ablaufplan eines Verfahrens zum Betreiben eines Visualisierungssystems.

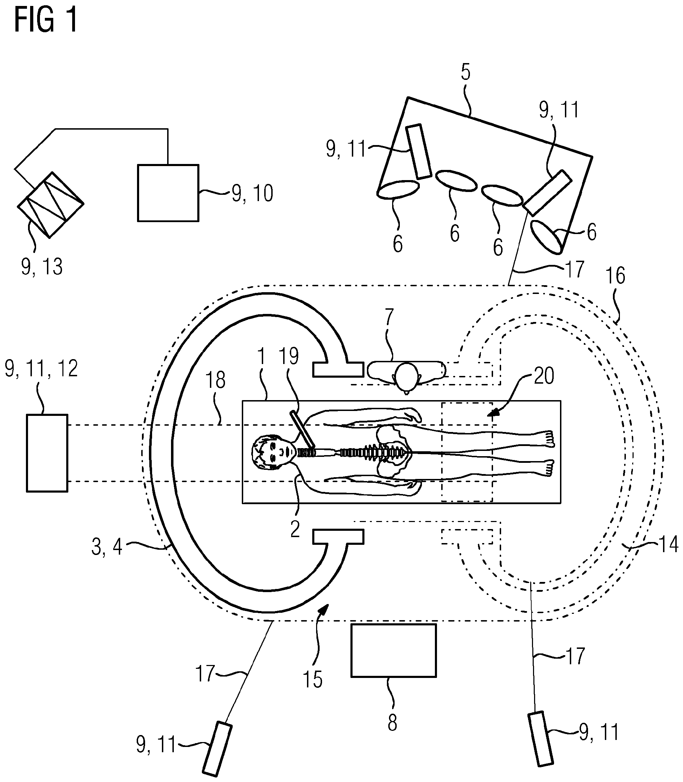

FIG 1 zeigt eine schematische Draufsicht auf eine Behandlungssituation, beispielsweise in einem Operationssaal (OP). Zentral ist hier eine Patientenliege beziehungsweise ein Patientenlager 1 mit einem darauf liegenden Patienten 2 dargestellt. Zur Untersuchung des Patienten 2 wird ein C-Bogen-Röntgengerät 3 verwendet, welches hier nur teilweise beziehungsweise angedeutet dargestellt ist. Das C-Bogen-Röntgengerät 3 kann beispielsweise mittels eines Mehrachsroboterarms gehalten sein und bewegt werden. Daher kann vorliegend auch das C-Bogen-Röntgengerät 3 als Roboter beziehungsweise als Teil eines Roboters aufgefasst werden. Vorliegend befindet sich das C-Bogen-Röntgengerät 3 in einer ersten Stellung 4.

Weiterhin dargestellt ist eine Leuchte 5, welche mehrere inkohärente Lichtquellen 6 zum Beleuchten des Patienten 2 aufweist. Neben dem Patientenlager befindet sich zudem eine Person 7, bei welcher es sich beispielsweise um einen Arzt oder sonstiges medizinisches Personal handeln kann. Die Person 7 kann hier insbesondere stellvertretend für weiteres, der Übersichtlichkeit halber nicht dargestelltes Personal verstanden werden. Hier beispielhaft auf einer gegenüberliegenden Seite des Patientenlagers 1 dargestellt ist ein mögliches Hindernis 8. Dabei kann es sich beispielsweise um einen Tisch, einen beweglichen medizinischen Trolley oder ein sonstiges Gerät oder Objekt handeln.

Soll nun während der Behandlung des Patienten 2 das C-Bogen-Röntgengerät 3 aus der ersten Stellung 4 in eine andere Stellung bewegt werden, um beispielsweise einen anderen Bereich des Patienten 2 abzubilden, so muss aus Sicherheitsgründen dafür Sorge getragen werden, dass es bei dieser Bewegung zu keiner Kollision, beispielsweise zwischen dem C-Bogen-Röntgengerät und der Person 7 und/oder dem möglichen Hindernis 8 kommt.

Hierfür ist vorliegend ein Visualisierungssystem 9 vorgesehen, von dem hier mehrere verteilt angeordnete Komponenten schematisch dargestellt sind. Anders als hier der Übersichtlichkeit halber gezeichnet, können die einzelnen Komponenten des Visualisierungssystems 9 bei einem realen Visualisierungssystem 9 direkt oder indirekt miteinander verbunden sein, beispielsweise über jeweilige elektrische Verbindungen und/oder Datenverbindungen. Vorliegend als Teil des Visualisierungssystems 9 dargestellt sind eine Datenverarbeitungseinrichtung 10, mehrere Laserlichtquellen 11 und eine 3D-Kamera 13. Vorliegend sind einige der Laserlichtquellen 11 in der Leuchte 5 angeordnet, während andere beispielsweise an einer Decke des Operationssaals angeordnet sein können, um eine möglichst ungehinderte oder verdeckungsfreie Sichtlinie auf das Patientenlager 1 und dessen Umgebung zu haben. Vorteilhaft kann eine Laserlichtquelle 12 der Laserlichtquellen 11 beispielsweise an einer Wand des Operationssaals in Hochrichtung des Raumes zumindest im Wesentlichen auf Höhe des Patienten 2, also beispielsweise etwa 10-20 cm oberhalb einer Oberfläche oder Haupterstreckungsebene des Patientenlagers 2, angeordnet sein.

Die 3D-Kamera 13 ist vorliegend so angeordnet, also ausgerichtet, dass sie eine Umgebung des Patientenlagers 1, insbesondere einschließlich des C-Bogen-Röntgengeräts 3 erfasst, also räumlich abbilden kann. Entsprechende Umgebungsdaten werden von der 3D-Kamera 13 an die Datenverarbeitungseinrichtung 10 übermittelt beziehungsweise dieser bereitgestellt. Dazu kann die Datenverarbeitungseinrichtung 10 beispielsweise eine entsprechende Schnittstelle oder Erfassungseinrichtung aufweisen. Ebenfalls mittels dieser Erfassungseinrichtung erfasst werden können beispielsweise eine Benutzereingaben oder ein Steuersignal, insbesondere eine Vorgabe betreffend eine Bewegung des C-Bogen-Röntgengeräts 3. Diese Bewegung kann insbesondere eine geplante und/oder maximal mögliche Bewegung des C-Bogen-Röntgengeräts 3 sein. Auch Steuer-, Konfigurations- und/oder Zustandsdaten des C-Bogen-Röntgengeräts 3 können an die Datenverarbeitungseinrichtung 10 übermittelt beziehungsweise von deren Erfassungseinrichtung empfangen oder erfasst werden.

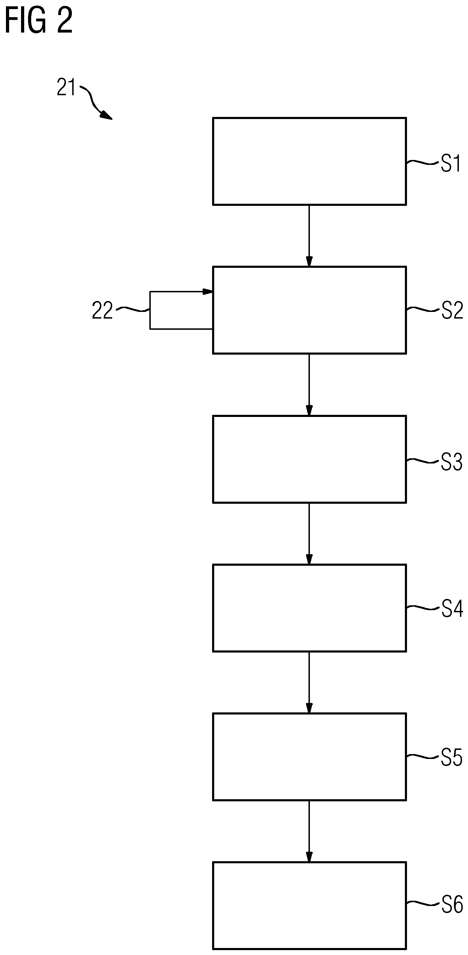

FIG 2 zeigt schematisch einen beispielhaften Ablaufplan 21 für ein Verfahren zum Betreiben eines Visualisierungssystems, insbesondere des Visualisierungssystems 9. Das Verfahren wird im Folgenden unter Bezugnahme auf FIG 1 und FIG 2 erläutert.

In einem Verfahrensschritt S1 wird das Verfahren gestartet, wozu beispielsweise das C-Bogen-Röntgengerät 3 und das Visualisierungssystem 9 in Betrieb genommen werden können.

In einem - optionalen - Verfahrensschritt S2 wird mittels der 3D-Kamera 13 die aktuelle Situation, also insbesondere das C-Bogen-Röntgengerät 3 und das Patientenlager 1, räumlich erfasst. Die 3D-Kamera 13 kann insbesondere auch während nachfolgender Verfahrensschritte kontinuierlich zum Erfassen der entsprechenden Umgebungsdaten betrieben werden, was hier schematisch durch eine Schleife 22 angedeutet ist. Die von der 3D-Kamera 13 bereitgestellten Umgebungsdaten können durch die Datenverarbeitungseinrichtung 10 beispielsweise mittels eines Bildverarbeitungs- und/oder Objekterkennungsalgorithmus verarbeitet werden. So können beispielsweise das C-Bogen-Röntgengerät 3, dessen aktuelle erste Stellung 4, das Patientenlager 1, der Patient 2, die Leuchte 5, die Person 7 und/oder das mögliche Hindernis 8 erkannt beziehungsweise identifiziert werden. Insbesondere können, da die 3D-Kamera 13 eine räumliche Erfassung ermöglicht, auch eine jeweilige Position und/oder jeweilige räumliche Relationen zwischen den bekannten oder erfassten Objekten bestimmt werden. Diese Daten können beispielsweise durch die Datenverarbeitungseinrichtung 10 zu einem dreidimensionalen digitalen Modell verarbeitet werden. Alternativ oder zusätzlich zum Einsatz der 3D-Kamera können beispielsweise bekannte Geräte, Gerätebestandteile und/oder Einrichtungsdetails modelliert und/oder visualisiert werden. Entsprechende Modelldaten über in dem Raumbereich und/oder dessen Umgebung befindliche Gegenstände können beispielsweise bereitgestellt oder, insbesondere automatisch, durch das Visualisierungssystem aus einer Datenbank abgerufen werden.

In einem Verfahrensschritt S3 wird dann von der Erfassungseinrichtung der Datenverarbeitungseinrichtung 10 beziehungsweise des Visualisierungssystems 9 eine Vorgabe für eine geplante Bewegung oder Fahrt des C-Bogen-Röntgengeräts 3 aus der ersten Stellung 4 in eine hier angedeutete zweite Stellung 14 erfasst.

In einem Verfahrensschritt S4 wird mittels der Datenverarbeitungseinrichtung 10 von dem Visualisierungssystem 9 automatisch ein Raumbereich 15 bestimmt oder ermittelt, der bei einer Ausführung der Bewegung oder Fahrt des C-Bogen-Röntgengeräts 3 gemäß der erfassten Vorgabe von dem C-Bogen-Röntgengerät 3 durchlaufen oder überfahren wird, demzufolge also für ein ungehindertes Bewegen des C-Bogen-Röntgengeräts freizuhalten ist. Auch kann hier beispielsweise eine Strahlendosis beziehungsweise eine Strahlenbelastung zumindest für den Raumbereich 15 berechnet werden, die entsteht, wenn das C-Bogen-Röntgengerät 3 während der Fahrt in die zweite Stellung 14 aktiv betrieben wird, also Röntgenstrahlung aussendet. Zum Bestimmen des Raumbereiches 15 können beispielsweise ein Modell und/oder eine Spezifikation des C-Bogen-Röntgengeräts 3 und/oder das von der Datenverarbeitungseinrichtung 10 erstellte Modell verwendet beziehungsweise verarbeitet werden. So kann zum Bestimmen des Raumbereiches 15 beispielsweise die Bewegung des C-Bogen-Röntgengeräts 3 gemäß der erfassten Vorgabe simuliert werden.

In einem Verfahrensschritt S5 wird der bestimmte Raumbereich 15 mittels einer Markierungseinrichtung des Visualisierungssystems 9 optisch markiert. Diese Markierungseinrichtung umfasst vorliegend beispielsweise zumindest die Laserlichtquellen 11. Zum Markieren des Raumbereiches 15 kann beispielsweise ein Umriss 16 kontinuierlich oder periodisch mittels von einer, einigen oder allen der Laserlichtquellen 11 ausgesendeten Laserstrahlen 17 beleuchtet beziehungsweise abgescannt werden. Der Umriss 16 kann beispielsweise einer orthogonalen Projektion eines von dem C-Bogen-Röntgengerät 3 oder einem Teil von diesem bei der Bewegung von der ersten Stellung 4 in die zweite Stellung 14 durchfahrenen oder überstrichenen Raumvolumens auf einen Boden des Operationssaals entsprechend.

Zusätzlich oder alternativ kann beispielsweise die Datenverarbeitungseinrichtung 10 ein Head-Mounted Display (HMD) der Person 7 ansteuern, sodass dieses in einer virtuellen Realität oder als Teil einer Augmented-Reality-Darstellung den Raumbereich 15 und/oder das entsprechende Raumvolumen anzeigt beziehungsweise visualisiert. Ein Beispiel für ein derartiges HMD kann beispielsweise die unter dem Namen Microsoft Holo-Lens bekannte Mixed-Reality-Brille der Microsoft Corporation sein.

Da nun also der Raumbereich 15 bestimmt ist, kann dieser automatisch auf Hindernisse, die eine Kollisionsgefahr für das C-Bogen-Röntgengerät 3 darstellen, überwacht werden. Vorliegend befindet sich beispielsweise die Person 7 zumindest teilweise in dem Raumbereich 15 und könnte demzufolge mit dem C-Bogen-Röntgengerät 3 kollidieren. Aufgrund der Markierung des Raumbereiches 15 kann die Person 7 diese Gefahr besonders einfach erkennen und sich selbstständig rechtzeitig aus dem Raumbereich 15 herausbewegen, um eine Kollision zu vermeiden.

Bei der Person 7 kann es sich beispielsweise um eine Bedienperson des C-Bogen-Röntgengeräts 3 handeln, welche die Vorgabe für die Fahrt in die zweite Stellung 14 gemacht hat und deren tatsächliche Auslösung oder Durchführung beispielsweise durch eine entsprechende Bedienhandlung, etwa eine Bedienung eines Hand- oder Fußschalters, veranlassen kann. Aufgrund der Markierung des Raumbereiches 15 kann die Person 7 dann besonders einfach und eindeutig erkennen, dass in dem vorliegenden Beispiel das mögliche Hindernis 8 sich tatsächlich außerhalb des Raumbereiches 15 befindet, sodass das C-Bogen-Röntgengerät 3 mit voller Geschwindigkeit bewegt werden kann, ohne dass es zu einer Kollision mit dem möglichen Hindernis 8 kommt. Aber selbst wenn das mögliche Hindernis 8 in den Raumbereich 15 hineinragen würde, könnte die Person 7 diese aufgrund der Markierung besonders einfach erkennen und müsste sich vorteilhaft beispielsweise nicht auf ihr Augenmaß verlassen. Hierdurch kann beispielsweise ein gegebenenfalls Zeit aufwändiges und vorliegend unnötiges Wegbewegen des möglichen Hindernisses 8 und/oder ein unnötig langsames und vorsichtiges Bewegen des C-Bogen-Röntgengerätes 3 vermieden werden.

Als Teil der Bewegung in die zweite Stellung 14 oder im Rahmen einer weiteren Bewegung des C-Bogen-Röntgengerätes 3 kann dieses beispielsweise näher als hier dargestellt an dem Patienten 2 entlang geführt werden. Für einen solchen Fall kann beispielsweise mittels der Laserlichtquelle 12 Laserlicht entlang einer Hüllfläche 18 ausgestrahlt werden. Diese Hüllfläche 18 kann sich beispielsweise in einem Abstand zu dem Patienten 2 erstrecken, der einem minimalen Abstand zwischen dem C-Bogen-Röntgengerät 3 und dem Patienten 2 bei der entsprechenden Bewegung des C-Bogen-Röntgengeräts 3 entspricht.

Vorliegend kann beispielsweise ein medizinisches Objekt 19 an der Wirbelsäule des Patienten 2 befestigt sein und aus dem Patienten 2 herausragende. Ragt das medizinische Objekt 19 soweit von dem Patienten 2 ab, dass es mit dem C-Bogen-Röntgengerät 3 kollidieren würde, so durchragt es auch die Hüllfläche 18 und wird somit von der Laserlichtquelle 12 angestrahlt. Dies ist besonders einfach und eindeutig beispielsweise für die Person 7 erkennbar. In entsprechender Art und Weise kann beispielsweise auch ein Arm oder ein Bein des Patienten 2 und/oder eine Lagerungshilfe die Hüllfläche 18 durchragen. In einem solchen Fall können das medizinische Objekt 19 beziehungsweise der Patient 2 in eine Stellung umgelagert werden, in der keine Kollisionsgefahr besteht. Dass keine Kollisionsgefahr besteht kann dabei aufgrund der entsprechend vorhandenen oder nicht vorhandenen Anstrahlung durch die Laserlichtquelle 12 besonders einfach überprüft und sichergestellt werden. So kann die Untersuchung des Patienten besonders schnell und effizient mit einer minimal notwendigen Anzahl von Bewegungen des C-Bogen-Röntgengeräts 3 und/oder des Patienten 2 durchgeführt werden.

Ebenso kann die Vorgabe beispielsweise eine maximal mögliche Bewegung des C-Bogen-Röntgengeräts 3 betreffen. Dann kann beispielsweise ein abbildbarer Bereich 20 optisch mittels der Laserlichtquellen 11 markiert werden, der bei einer maximalen Verstellung, also bei maximaler Ausnutzung eines möglichen Bewegungsumfangs des C-Bogen-Röntgengeräts 3, von diesem durchleuchtet beziehungsweise abgebildet werden kann. So kann also ohne Testaufnahmen und daher mit minimaler Strahlenbelastung des Patienten 2 bereits vor dem Bewegen des C-Bogen-Röntgengeräts 3 in die entsprechende Stellung sichergestellt beziehungsweise überprüft werden, ob ein bestimmter Teilbereich des Patienten 2 in dessen aktueller Position relativ zu dem C-Bogen-Röntgengerät 3 abgebildet werden kann.

In einem Verfahrensschritt S6 kann beispielsweise die Person 7 die Bewegung des C-Bogen-Röntgengeräts 3 veranlasst haben. Es kann dabei möglich sein, dass auch nach erfolgter Freigabe der Bewegung durch die Person 7 die Bewegung erst dann tatsächlich ausgeführt wird, wenn die Überwachung des Raumbereiches 15 auf Hindernisse ergibt, dass aktuell keine Kollisionsgefahr besteht. Ebenso ist es möglich, dass die Bewegung des C-Bogen-Röntgengeräts 3 verlangsamt, gestoppt oder angepasst wird, wenn ein Hindernis in dem Raumbereich 15 detektiert wird. Die Datenverarbeitungseinrichtung 10 beziehungsweise das Visualisierungssystem 9 kann also beispielsweise ein entsprechendes Freigabesignal oder ein entsprechendes Steuersignal an das C-Bogen-Röntgengerät 3 beziehungsweise an dessen Steuergerät übermitteln. Ebenso können dieses Steuergerät und die Datenverarbeitungseinrichtung 10 beispielsweise in einem einzigen Gerät kombiniert sein. Zusätzlich oder alternativ kann beispielsweise durch das Visualisierungssystem 9 eine Warnung ausgegeben werden, wenn ein Hindernis in dem Raumbereich 15 detektiert wird.

## Patentansprüche

1. Visualisierungssystem (9) zum Anzeigen eines Raumbereiches (15, 18, 20), mit einer
- Erfassungseinrichtung zum Erfassen einer Vorgabe für eine geplante oder maximal mögliche Bewegung eines Roboters (3), und mit einer
- von der Erfassungseinrichtung in Abhängigkeit von der Vorgabe angesteuerten Markierungseinrichtung (10, 11, 12) zum optischen Markieren des Raumbereiches 15, 18, 20), der von dem Roboter (3) bei der Bewegung gemäß der Vorgabe erreichbar ist.

2. Visualisierungssystem (9) nach Anspruch 1, **dadurch gekennzeichnet, dass** die Markierungseinrichtung (10, 11, 12) eine Leuchte (5) aufweist, in der eine Lichtquelle (6) zur flächigen Ausleuchtung eines Arbeitsbereiches des Roboters (3) mit inkohärentem Licht und eine Laserlichtquelle (11) zum Markieren des Raumbereiches (15, 18, 20) angeordnet sind.

3. Visualisierungssystem (9) nach einem der vorhergehenden Ansprüche, **dadurch gekennzeichnet, dass** die Markierungseinrichtung (10, 12) eine Laserlichtquelle (12) zum zumindest bereichsweisen Einhüllen (18) eines Zielobjekts (2) des Roboters (3) mittels von der Laserlichtquelle (12) ausgestrahltem Laserlicht aufweist.

4. Visualisierungssystem (9) nach einem der vorhergehenden Ansprüche, **dadurch gekennzeichnet, dass** die Markierungseinrichtung (10, 11, 12) eine Darstellungseinrichtung, insbesondere ein Head-Mounted Display, zum Visualisieren des Raumbereiches (15, 18, 20) mittels einer auf augmentierter Realität basierenden Darstellung aufweist.

5. Visualisierungssystem (9) nach einem der vorhergehenden Ansprüche, **dadurch gekennzeichnet, dass** das Visualisierungssystem (9)
- eine 3D-Kamera (13) zum räumlichen Erfassen einer, insbesondere den Raumbereich (15, 18, 20) umfassenden, Umgebung des Roboters (3) aufweist, und
- eingerichtet ist zum Markieren des Raumbereiches (15, 18, 20) in Abhängigkeit von den von der 3D-Kamera (13) bereitgestellten Umgebungsdaten.

6. Verfahren zum Betreiben eines Visualisierungssystems (9), bei dem
- eine Vorgabe für eine geplante oder für eine maximal mögliche Bewegung eines Roboters (3) erfasst wird, und
- ein Raumbereich (15, 18, 20), der von dem Roboter (3) bei der Bewegung erreichbar ist, optisch markiert wird.

7. Verfahren nach Anspruch 6, **dadurch gekennzeichnet, dass** als der Raumbereich (15, 18, 20) ein für eine ungehinderte Bewegung des Roboters (3) freizuhaltender Raumbereich (15, 18, 20) markiert wird, der von dem Roboter (3) bei der Bewegung von einer aktuellen Istposition (4) hin zu einer vorgegebenen Zielposition (14) durchlaufen und/oder überfahren wird.

8. Verfahren nach einem der Ansprüche 6 und 7, **dadurch gekennzeichnet, dass** als der Raumbereich (20) ein von dem als medizinisches bildgebendes Gerät ausgebildeten Roboter (3), insbesondere bei Ausnutzung des maximal möglichen Bewegungsumfangs des Roboters (3), abbildbarer Bereich (20) eines Untersuchungsobjekts (1, 2) optisch markiert wird.

9. Verfahren nach einem der Ansprüche 6 bis 8, **dadurch gekennzeichnet, dass**
- in Abhängigkeit von der Vorgabe eine bei der entsprechenden Bewegung des als medizinisches bildgebendes Gerät ausgebildeten Roboters (3) auftretende räumliche Verteilung einer Strahlendosis berechnet wird, und
- in dem Raumbereich (15, 18, 20) und/oder in einer Umgebung des Raumbereiches (15, 18, 20) die jeweils berechnete Strahlendosis visualisiert wird, insbesondere durch unterschiedliche Kennzeichnung von Zonen, in denen die jeweilige Strahlendosis in unterschiedlichen vorgegebenen Werteintervallen liegt.

10. Verfahren nach einem der Ansprüche 6 bis 9, **dadurch gekennzeichnet, dass**
- eine den Raumbereich (15, 18, 20) umfassende Umgebung des Roboters (3) automatisch räumlich erfasst und auf ein Vorhandensein von Hindernissen (7, 8) hin überwacht wird, und
- wenn ein Hindernis (7, 8) in dem Raumbereich (15, 18, 20) oder in der Umgebung detektiert wird, automatisch eine Warnung ausgegeben wird und/oder die Bewegung des Roboters (3) in Abhängigkeit von einer detektierten Position des Hindernisses (7, 8) angepasst wird.
